# FASCICULE DE BREVET EUROPEEN

(11) **EP 0 717 031 B1**
(45) Date de publication et mention de la délivrance du brevet: **15.04.1998**
(21) Numéro de dépôt: 95402777.7
(22) Date de dépôt: 11.12.1995
(51) Int. Cl.: C07C 67/60, C07C 69/54

(54) **Procédé de récupération des produits nobles légers contenus dans les résidus de distillation des procédés de fabrication de l'acide acrylique et de ses esters**
Verfahren zur Gewinnung von leichten Wertprodukten aus den Destillationsrückständen der Verfahren zur Herstellung von Acrylsäure und ihren Estern
Process for recovering the light noble products contained in the distillation residues from the processes of production of acrylic acid and its esters

(30) Priorité: 12.12.1994 FR 9414935
(43) Date de publication de la demande: 19.06.1996
(73) Titulaire: ELF ATOCHEM S.A., 92800 Puteaux (FR)
(72) Inventeur: Fauconet, Michel, F-57730 Valmont (FR); Richard, Norbert, F-57500 Saint Avold (FR); Delafin, Patrick, F-04600 Saint Auban (FR)
(74) Mandataire: Chaillot, Geneviève

(56) Documents cités:
- FR-A- 1 307 204
- FR-A- 2 194 681
- US-A- 3 086 046

## Description

La présente invention porte sur un procédé de récupération des produits nobles légers contenus dans les résidus de distillation des procédés de fabrication de l'acide acrylique et des esters dudit acide et d'alcools en C₁-C₈, par dissociation thermique (ou craquage) desdits résidus. Par produits nobles, on entend l'acide acrylique monomère, lesdits esters acryliques monomères et lesdits alcools.

Dans les procédés de synthèse de l'acide acrylique, utilisant par exemple l'oxydation catalytique du propylène, on obtient des sous-produits lourds, qui sont élimines en pied des dernières colonnes de distillation. Ces mélanges de composés lourds contiennent principalement les dérivés 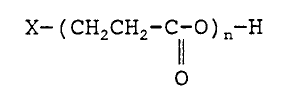 n étant supérieur ou égal à 1,
générés par des réactions de condensation, sur la double liaison de l'acide acrylique :
- de l'acide acrylique lui-même 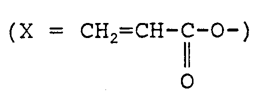
- de l'acide acétique 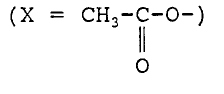
- de l'eau (X = HO-)

De même, dans les procédés de synthèse des esters à partir de l'acide acrylique, les produits lourds éliminés en pied des colonnes de distillation contiennent les esters correspondants de ces composés avec l'alcool utilisé lors des synthèses de ces esters, ainsi que les produits de condensation des alcools (X = RO-, R = alkyle en C₁-C₈) sur les doubles liaisons des composés précédents.

En outre, on retrouve, dans ces mélanges de produits lourds, une partie des inhibiteurs de polymérisation utilisés dans les procédés.

Ces mélanges de produits lourds peuvent être évaporés pour récupérer l'essentiel des monomères (acide acrylique et esters) qu'ils contiennent. En l'absence d'un procédé approprié pour les valoriser, les résidus obtenus sont alors perdus, ce qui représente une quantité importante de déchets qui sont difficiles à éliminer, et également une perte de monomères et d'alcools.

La récupération de monomères par dissociation thermique en phase liquide des dérivés d'addition sur la double liaison acrylique est bien décrite dans la littérature.

Le problème principal de ce type de traitement de dissociation thermique, appliqué à un mélange réel de produits lourds obtenus en pied des dernières colonnes de distillation de l'acide acrylique, est la viscosité importante du résidu, qui devient non véhiculable dans les conduites. Cette viscosité excessive s'accompagne d'un dépôt de goudrons insolubles qui provoque un encrassement de la paroi du réacteur de craquage, entraînant une diminution d'efficacité du transfert de chaleur à travers la paroi, avec, pour conséquence finale, une réduction du rendement de craquage. Ces phénomènes d'encrassement obligent à des nettoyages périodiques fréquents qui rendent incompatible ce mode de fonctionnement en régime continu et réduisent la productivité en régime discontinu.

La viscosité du résidu obtenu à l'issue du craquage augmente avec le temps de séjour à haute température du mélange de produits lourds à traiter et avec la quantité de monomères légers récupérés par distillation. Pour obtenir une viscosité du résidu compatible avec les conditions de transfert normales des flux liquides et réduire les phénomènes d'encrassement, on est contraint de limiter ces deux paramètres, ce qui a pour effet de réduire le rendement de craquage.

Ces inconvénients sont réduits lorsque le traitement est appliqué à la récupération de produits nobles dans les lourds issus de la fabrication des esters acryliques, comme il est décrit dans le brevet français FR-B-2 194 681.

Les procédés qui sont relatés pour la récupération de produits nobles dans les produits lourds de fabrication de l'acide acrylique sont essentiellement catalytiques. Les catalyseurs décrits sont très variés : carboxylates, phosphates, borates (FR-A-2 261 252) ; acides, tels qu'H₂SO₄ (JP-05 025086) ; alumine ou silice (JP-75 69014) ; amines secondaires, tertiaires ou phosphines (FR-1 572 316) ; acides solides tels que zéolithes (JP-03 178 949) ; catalyseurs de type Friedel et Crafts (US-2 806 878) ; et titanates d'alcoxydes (JP-06 0655149).

Outre le fait que ces procédés ne résolvent pas le problème crucial de la limitation du rendement de récupération à cause de la viscosité excessive des résidus et de la formation de goudrons indésirables, d'autres inconvénients sont plus particulièrement liés à l'utilisation des catalyseurs :
- coût du catalyseur ;
- phénomènes de corrosion ;
- difficulté d'élimination dans les résidus lourds, d'où un coût élevé de traitement des déchets :
   - déchets inorganiques difficiles à incinérer ;
   - présence de résidus métalliques provoquant l'encrassement des fours d'incinération ;
   - élimination par lavages aqueux, entraînant une augmentation de la pollution rejetée dans les eaux usées ;
- problèmes de désactivation des catalyseurs solides, dus à l'encrassement des pores catalytiques actifs par dépôts de composés partiellement polymérisés.

Pour réduire ces inconvénients, le brevet US-3 086 046 décrit un traitement non catalytique consistant en une évaporation du mélange à craquer, suivie par une dissociation thermique en phase vapeur, à très haute température (350-650 °C) sous une pression de 6,7 x 10² - 2 x 10⁴ Pa (5-150 mmHg). Ce procédé, adapté au traitement de mélanges très riches en produits à faible masse moléculaire (acide acrylique et dimère de l'acide acrylique), n'est pas utilisable dans le cas d'un résidu lourd réel de distillation, obtenu par exemple dans le cadre du procédé de fabrication de l'acide acrylique à partir de propylène et d'oxygène. La fraction du mélange vaporisée en première étape serait, dans ce cas, réduite par la faible quantité de produits à points d'ébullition suffisamment bas, ou l'augmentation de cette fraction nécessiterait une température de vaporisation telle que le problème de la forte viscosité des résidus éliminés en pied de vaporiseur deviendrait à nouveau une limitation.

L'extraction à l'eau de l'acide acrylique contenu dans le milieu réactionnel de dissociation thermique est revendiquée par plusieurs brevets : FR-A-2 415 092, JP-57 062 229, lequel utilise une solution aqueuse basique, et JP-56 147 745, lequel décrit un procédé par addition d'eau suivie d'extraction par un solvant organique. L'inconvénient majeur de ces procédés est que le ou les monomères régénérés ne sont pas éliminés du milieu, au fur et à mesure de leur formation, pendant la réaction de dissociation thermique. De ce fait, on ne peut éviter une recombinaison instantanée (réaction inverse) des dérivés d'addition ou de polymérisation à partir des produits dissociés présents dans ce milieu.

Une autre façon de réduire le problème de l'excès de viscosité des résidus lourds obtenus par craquage thermique des lourds de fabrication de l'acide acrylique serait d'utiliser un solvant. L'inconvénient d'un tel procédé est qu'il nécessiterait une récupération coûteuse du solvant utilisé.

De manière surprenante, la Société déposante a découvert que le fait d'opérer la dissociation thermique ou craquage des produits lourds de la synthèse de l'acide acrylique en présence de produits lourds récupérés en pied des dernières colonnes de fabrication des esters acryliques, avec distillation instantanée des produits légers générés, permet d'améliorer sensiblement leur rendement de récupération, en réduisant les encrassements dans le réacteur de dissociation et la viscosité du résidu obtenu à l'issue de l'opération de dissociation thermique.

La présente invention a donc pour objet un procédé tel que défini dans l'introduction de cette description, ce procédé étant caractérisé par le fait qu'on opère ce craquage, en l'absence de catalyseur, sur un mélange de résidus lourds de distillation issus, d'une part, de la fabrication de l'acide acrylique, et d'autre part, de la fabrication des esters précités dudit acide, qu'on vaporise continuellement, pendant l'opération de craquage, la fraction légère issue des réactions de dissociation, et qu'on distille cette fraction légère afin de récupérer, après condensation, les produits nobles légers recherchés.

Dans le mélange des résidus soumis au procédé de l'invention, le rapport massique des résidus lourds issus du procédé de fabrication de l'acide acrylique aux résidus lourds issus du procédé de fabrication d'un ester de l'acide acrylique est avantageusement compris entre 9/1 et 1/9.

Le craquage est avantageusement conduit à une température comprise entre 140 et 260°C, de préférence entre 180 et 220°C, avec un temps de séjour de 0,5 à 3 heures.

La distillation de la fraction légère vaporisée est avantageusement conduite sous pression réduite, et également avantageusement en présence d'au moins un inhibiteur de polymérisation, le ou les inhibiteurs de polymérisation pouvant être introduits via l'introduction d'une partie du flux alimentant la colonne de distillation.

Conformément à une caractéristique intéressante du procédé selon l'invention, on recycle les produits nobles légers obtenus à l'unité de fabrication d'ester de laquelle sont issus les résidus lourds utilisés conjointement avec les résidus lourds issus de la fabrication de l'acide acrylique.

Conformément à un premier mode de réalisation du procédé selon l'invention, conduit en continu, on effectue les opérations consistant à :
- conduire le craquage du mélange des résidus lourds à craquer, éventuellement préchauffé, dans un réacteur (R1) ;
- alimenter une colonne de distillation (C1) par les produits légers du craquage ;
- récupérer, en tête de colonne (C1), le mélange de produits nobles légers recherché ;
- recycler dans le réacteur (R1) le flux récupéré en pied de la colonne (C1) ;
- récupérer le résidu lourd du réacteur (R1) et l'adresser à un traitement d'élimination, le cas échéant après l'avoir dilué avec une partie du flux récupéré en pied de la colonne (C1), le reste dudit flux étant recyclé dans le réacteur (R1).

Conformément à un second mode de réalisation de la présente invention, le mélange de produits lourds mis en jeu est distillé, simultanément avec les légers récupérés à l'issue de l'étape de craquage et le réacteur de craquage est alimenté par le mélange de lourds obtenu en pied de colonne de distillation. Ce mode de fonctionnement est particulièrement adapté au traitement de lourds riches en produits légers non combinés (acide acrylique, esters monomères et alcools). Conduit en continu, il comprend les opérations consistant à :
- adresser à une colonne de distillation (C'1) le mélange des résidus lourds à craquer ;
- récupérer, en tête de colonne (C'1), un mélange de produits nobles légers ;
- adresser à un réacteur de craquage (R'1) le flux obtenu en pied de la colonne (C'1) ;
- recycler à la colonne de distillation (C'1) le mélange des produits légers du craquage ;
- récupérer le résidu lourd du réacteur (R'1) et l'adresser à un traitement d'élimination ultérieur, le cas échéant après l'avoir dilué avec une partie du flux récupéré en pied de la colonne (C'1), le reste dudit flux étant adressé au réacteur de craquage (R'1).

Pour mieux illustrer le procédé de la présente invention, on va en décrire plus en détail ci-après les deux modes de réalisation précités avec référence aux Figures respectivement 1 et 2, qui représentent chacune le schéma d'installation correspondant.

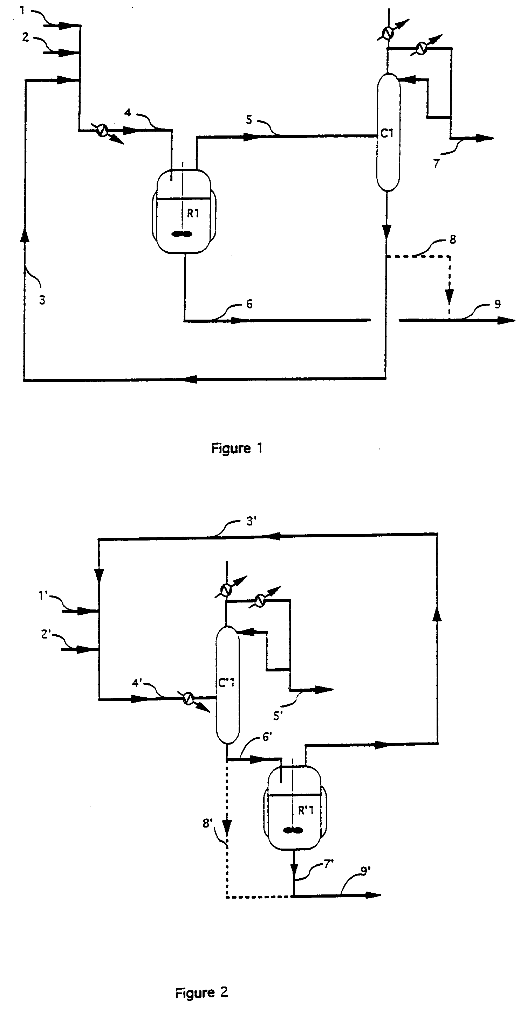

Si l'on se réfère tout d'abord à la Figure 1, on peut voir que l'on envoie dans le réacteur de craquage R1, via la conduite 4, un mélange de produits lourds issus de la fabrication de l'acide acrylique (conduite 1), de produits lourds issus de la fabrication de l'ester acrylique (conduite 2) et d'une partie du flux récupéré en pied de la colonne C1 (conduite 3). Ce mélange ainsi constitué est éventuellement préchauffé avant d'être envoyé dans le réacteur de craquage R1. Ce dernier est chauffé, et le mélange de produits légers obtenus est envoyé, via la conduite 5, en alimentation de la colonne C1, laquelle fonctionne de manière préférentielle sous une pression réduite. On envoie dans la partie supérieure de la colonne C1 un inhibiteur de polymérisation, choisi parmi les inhibiteurs classiques ou un mélange de ces inhibiteurs (phénothiazine, hydroquinone, phénols, éthers de phénols, etc...). La stabilisation de la partie supérieure de la colonne peut (avantageusement) être assurée grâce à l'introduction en tête d'une partie du flux alimentant la colonne. En tête de la colonne C1, on récupère, après condensation, un mélange de produits légers, dont une partie constitue le reflux. Le produit distillé, pratiquement exempt de produits lourds, est constitué essentiellement d'acide acrylique, d'ester acrylique monomère, d'alcool et d'eau. Ce mélange de produits nobles légers peut être avantageusement recyclé (conduite 7) en amont du procédé de fabrication de l'ester acrylique ayant fourni les lourds envoyés dans R1 par la conduite 2.

Le résidu obtenu en fond de réacteur R1 (conduite 6) peut être éventuellement dilué par une partie du flux récupéré en pied de colonne C1 (conduite 8). Le reste du flux obtenu en pied de colonne C1 est envoyé en alimentation du réacteur R1 (conduite 3). Le résidu lourd épuisé est envoyé à un traitement d'élimination classique ultérieur (conduite 9).

Si l'on se réfère maintenant à la Figure 2, on peut voir qu'en alimentation de la colonne C'1 (conduite 4'), on envoie le mélange de produits lourds issus de la fabrication de l'acide acrylique (conduite 1'), de produits lourds issus de la fabrication de l'ester acrylique (conduite 2'), et de tout ou partie du flux de légers récupéré en tête de réacteur de craquage R'1 (conduite 3').

La colonne C'1 fonctionne de manière préférentielle sous une pression réduite, et on envoie dans sa partie supérieure un inhibiteur de polymérisation, comme indiqué pour le premier mode de réalisation. Elle permet de séparer l'essentiel des produits lourds en pied de colonne. Le produit distillé est constitué essentiellement d'acide acrylique, d'ester acrylique monomère, d'alcool et d'eau. Ce mélange de produits nobles légers peut être avantageusement recyclé (conduite 5') en amont du procédé de fabrication de l'ester acrylique ayant fourni les lourds envoyés dans C'1 par la conduite 2'.

Le flux obtenu en pied de colonne C'1 est envoyé dans le réacteur de craquage R'1 (conduite 6'). Le réacteur R'1 est chauffé, et le mélange de produits légers vaporisé est envoyé en alimentation de la colonne C'1, via la conduite 3'. Le flux de résidu lourd épuisé provenant du réacteur de craquage R'1, via la conduite 7', peut être dilué, optionnellement, par une partie du flux collecté en pied de colonne C'1 (conduite 8'). Ce résidu lourd épuisé est envoyé à un traitement d'élimination classique ultérieur (conduite 9').

Les exemples suivants illustrent encore la présente invention. Dans ces exemples, les pourcentages sont exprimés en poids et les abréviations suivantes ont été utilisées :
- AA :: acide acrylique
- LAA:: fraction de produits lourds issue de la fabrication de l'AA
- AE :: acrylate d'éthyle
- LAE:: fraction de produits lourds issue de la fabrication de l'AE
- AM :: acrylate de méthyle
- LAM:: fraction de produits lourds issue de la fabrication de l'AM
- Dimère et trimère de l'AA :: composés de formules respectivement 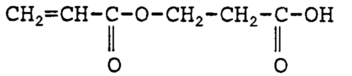 et 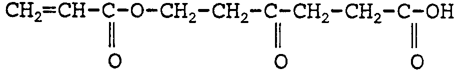 issus de la condensation de l'acide acrylique sur lui-même
- APE:: acryloxypropionate d'éthyle (ester éthylique du dimère de l'AA)
- APM:: acryloxypropionate de méthyle (ester méthylique du dimère de l'AA)
- EPE:: 2-éthoxypropionate d'éthyle (dérivé d'addition de l'éthanol sur la double liaison de l'AE)
- MPM:: 2-méthoxypropionate de méthyle (dérivé d'addition du méthanol sur la double liaison de l'AM).
- Taux de craquage :: rapport, exprimé en pourcentage massique, de la fraction légère récupérée pendant le craquage sur le mélange de lourds introduit dans le réacteur.
- Taux de récupération de l'AA et des esters :: rapport, exprimé en pourcentage massique, des monomères AA et esters récupérés dans la fraction distillée pendant l'opération de craquage, sur le mélange de lourds introduit dans le réacteur.

### Exemple 1

Dans un réacteur agité, d'une capacité de 0,5 litre, chauffé par bain d'huile, surmonté d'une colonne de faible hauteur pourvue de quelques pointes Vigreux faisant office de dévésiculeur, d'un condenseur et d'une recette, on introduit 200 g de mélanges de LAA et de LAE. On chauffe les mélanges à ébullition et on distille les produits légers générés par la réaction.

Le mélange de LAA est composé de 2,8% d'AA, 25,4% de dimère de l'AA, 9,8% d'hydroquinone, le complément étant formé essentiellement de produits de condensation plus lourds de l'AA.

Le mélange de LAE est constitué de 9,2% d'AA monomère, 8,8% de dimère de l'AA, 0,08% d'AE, 0,08% d'éthanol, 1,3% d'hydroquinone, le complément étant principalement composé d'APE, d'EPE et de produits de condensation plus lourds de l'AA ou de l'AE.

Les Tableaux 1 à 4 ci-après résument les résultats de plusieurs essais réalisés avec différents ratios LAA/LAE, pour différents taux de craquage. Les températures atteintes pendant les essais sont comprises entre 190°C et 250°C.

Les résultats sont exprimés par :
- la viscosité du résidu obtenu à l'issue de l'expérience de craquage. Cette viscosité est exprimée en centipoises (Pa.s) et est mesurée à une température de 100°C (Tableau 1). On remarque la très nette diminution de la viscosité du résidu obtenu après craquage, lorsqu'on utilise un mélange de LAA et de LAE par rapport à un craquage réalisé à partir de la fraction LAA seule ;
- le taux de récupération de l'AA et de l'AE (Tableaux 2 et 3).

Par extrapolation de ces résultats, on peut calculer les taux de récupération de l'AA et de l'AE pour une viscosité équivalente du résidu après craquage. Le Tableau 4 donne les résultats de ce calcul pour une viscosité du résidu de 500 Pa.s à 100°C.

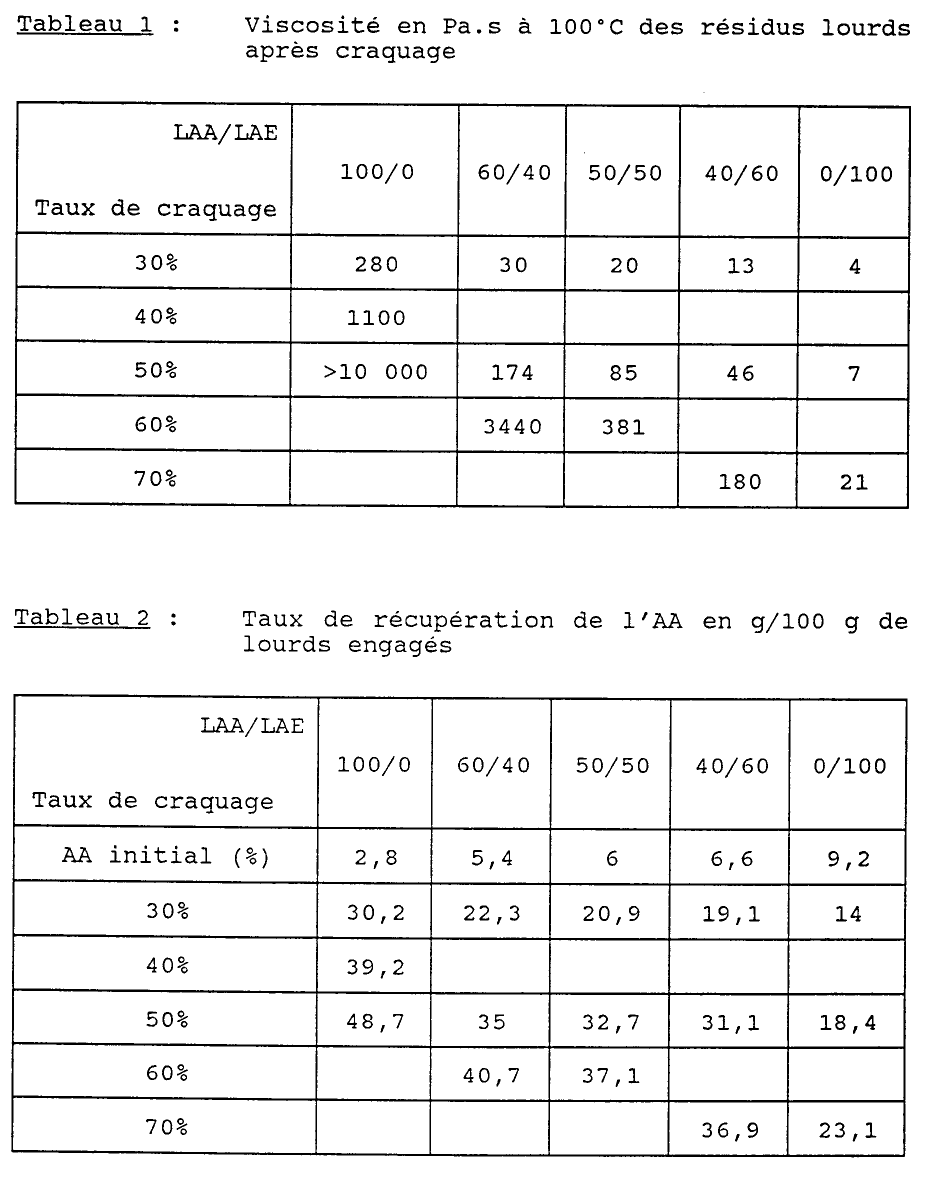

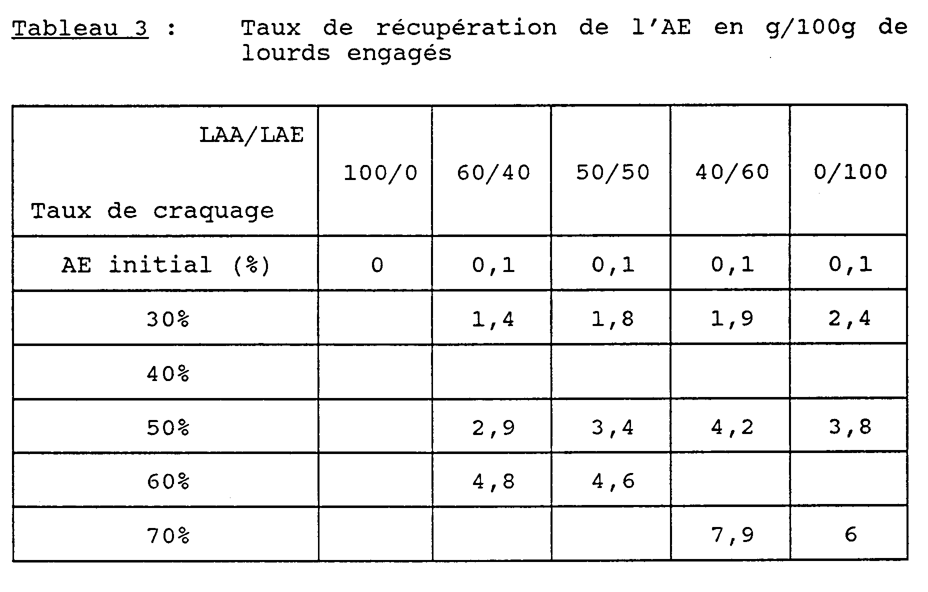

**Tableau 4**

| Taux de récupération extrapolé pour une viscosité du résidu de 500 Pa.s à 100°C | | | | |
|---|---|---|---|---|
| LAA/LAE | Taux de craquage 500 Pa.s (%) | Taux de récupération (%) | | |
| | | AA | AE | Somme |
| 100/0 | 35 | 35 | 0 | 35 |
| 60/40 | 56 | 38 | 3,8 | 41,8 |
| 50/50 | 62 | 37,5 | 4,8 | 42,3 |
| 40/60 | 66 | 36 | 7 | 43 |
| 0/100 | 85 | 26 | 8,3 | 34,3 |

Le Tableau 4 montre l'amélioration du taux de récupération de monomères par craquage de mélanges de LAA et de LAE par rapport à celui qui est obtenu avec soit la fraction LAA, soit la fraction LAE.

### Exemple 2

On opère de façon continue selon le procédé schématisé sur la Figure 1, dans un montage constitué comme décrit ci-après :

### (a) Partie réaction :

- une recette de préparation, dans laquelle est chargé le mélange de produits lourds à craquer, d'une capacité de 1 litre, équipée d'un agitateur mécanique, d'une double enveloppe chauffée par de l'huile à une température régulée à 90°C ; cette recette de préparation alimente en continu le réacteur de craquage ;
- un réacteur de craquage R1 d'une capacité de 0,5 litre, équipé d'un agitateur mécanique, d'une double enveloppe dans laquelle circule de l'huile à une température régulée, d'une sortie calorifugée en fond de réacteur pour éliminer le résidu de la réaction de craquage, d'une sonde de température. Ce réacteur est surmonté d'une colonne calorifugée de faible hauteur pourvue de pointes Vigreux pour éviter les entraînements de phase liquide (dévésiculeur) et d'un condenseur, après dévésiculeur, alimenté par de l'eau à 20°C ;
- une recette calorifugée, qui reçoit le résidu lourd obtenu en pied du réacteur de craquage R1 ;
- une recette qui reçoit le mélange de légers condensé en tête du réacteur R1.

### (b) Partie distillation :

- une colonne Vigreux adiabatique C1 d'efficacité 6 plateaux théoriques, alimentée en continu par le mélange de produits légers condensé en tête de réacteur R1, équipée :
   - d'un rebouilleur à thermosiphon en pied, alimenté dans sa paroi externe par de l'huile à température régulée ;
   - d'un condenseur en tête, alimenté par de l'eau à 20°C ;
   - d'une sonde de température en tête ;
   - d'un système de soutirage permettant de renvoyer une partie de la phase liquide condensée pour assurer un reflux en tête de colonne ;
   - d'un système de pompe à vide équipé d'une régulation, permettant de maintenir une pression réduite dans la colonne ;
   - d'une injection de phénothiazine en solution à 1% dans l'acide acrylique en tête de colonne;
- une recette qui reçoit le flux sortant en pied de colonne C1 ;
- une recette qui reçoit le mélange de produits légers distillés en tête de colonne C1.

Les conditions opératoires et résultats obtenus en partie réaction sont résumés dans le Tableau 5, pour divers mélanges de produits lourds à craquer :
- Mélange LAA/LAE = 50/50 ayant la composition suivante : AA monomère : 3,8% ; dimère de l'AA : 15,6% ; AE : 0,06%, éthanol : 0,3%, le complément étant formé essentiellement de produits de condensation plus lourds de l'AA ou de l'AE.
- Mélange LAA/LAM = 50/50 ayant la composition suivante : AA monomère : 1,3% ; dimère de l'AA : 9,4%, AM : 0,01%, méthanol : 0,09%, le complément étant formé essentiellement de produits de condensation plus lourds de l'AA ou de l'AM.
- LAA seul ayant la composition suivante : AA monomère : 1,8%, dimère de l'AA : 22,5% ; trimère de l'AA : 4,1% ; hydroquinone : 9,8% ; le complément étant formé essentiellement de produits de condensation plus lourds de l'AA.

**Tableau 5**

| Viscosité et taux de récupération pour différents mélanges de lourds - Réaction en continu | | | | | | | | |
|---|---|---|---|---|---|---|---|---|
| | | LAA/LAE = 50/50 | | LAA/LAM = 50/50 | | | LAA seul | |
| Température R1 | °C | 206 | 221 | 203 | 206 | 216 | 200 | 230 |
| Temps de séjour R1 | min. | 54 | 54 | 55 | 58 | 53 | 75 | 74 |
| Taux de craquage | % | 41 | 61 | 37 | 46 | 56 | 34 | 49 |
| Viscosité à 100°C | Pa.s | 34 | 1055 | 69 | 277 | 1285 | 4210* | >10 000* |

| Taux de récupération : | | | | | | | | |
|---|---|---|---|---|---|---|---|---|
| - AA | % | 25,5 | 34,8 | 22,1 | 27,5 | 29,9 | 31,4 | 42,5 |
| - ester | % | 2,4 | 3,7 | 2,8 | 4,4 | 4,3 | 0 | 0 |
| - alcool | % | 0,5 | 0,7 | 0,3 | 0,3 | 0,4 | 0 | 0 |

| | | | | | | | | |
|---|---|---|---|---|---|---|---|---|
| * : bouchage de la conduite d'élimination du résidu | | | | | | | | |

Là encore, on observe une réduction importante de la viscosité du résidu lourd obtenu en pied de réacteur R1, et une augmentation du taux de récupération pour une viscosité équivalente, après craquage thermique dans les mélanges de LAA et LAE et de LAA et LAM par rapport aux traitements réalisés à base de la fraction LAA seule. En particulier, les viscosités supérieures à 4000 Pa.s (mesurées à 100°C) obtenues lors du craquage de la fraction LAA seule sont trop importantes pour permettre un écoulement régulier du résidu obtenu.

Après cette étape de craquage thermique, les légers récupérés en tête de réacteur R1 sont distillés en continu sur le montage décrit précédemment. Les conditions opératoires et résultats obtenus sont résumés dans les Tableaux 6 et 7.

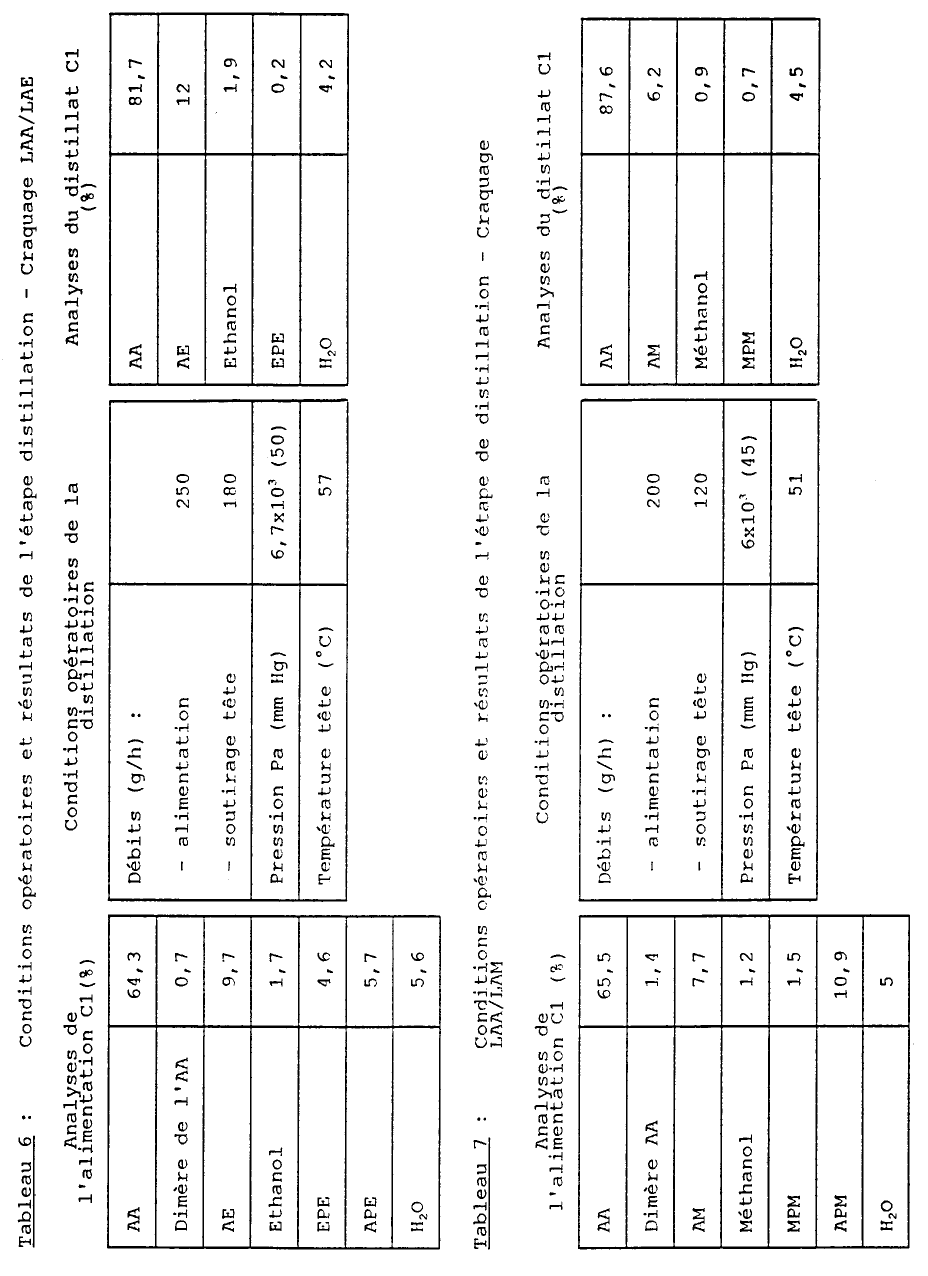

### Exemple 3

On opère de façon continue selon le procédé schématisé sur la Figure 2, dans un montage constitué comme décrit ci-après :

### (a) Partie distillation :

- une recette de préparation, d'une capacité de 1 litre, dans laquelle sont chargés le mélange de LAA et LAE à craquer, ainsi que les légers obtenus pendant la réaction de craquage. Cette recette est équipée d'un agitateur mécanique, d'une double enveloppe chauffée par de l'huile à une température régulée à 90°C ;
- une colonne Vigreux adiabatique C'1 d'efficacité 12 plateaux théoriques, alimentée en continu, en milieu de colonne, par le mélange contenu dans la recette de préparation, et équipée :
   - d'un rebouilleur à thermosiphon en pied, alimenté dans sa paroi externe par de l'huile à température régulée ;
   - d'un condenseur en tête, alimenté par de l'eau à 20°C ;
   - d'une sonde de température en tête ;
   - d'un système de soutirage permettant de renvoyer une partie de la phase liquide condensée pour assurer un reflux en tête de colonne ;
   - d'un système de pompe à vide équipé d'une régulation, permettant de maintenir une pression réduite dans la colonne ;
   - d'une injection de phénothiazine en solution à 1% dans l'AA en tête de colonne;
- une recette qui reçoit le flux sortant en pied de colonne C'1 ;
- une recette qui reçoit le mélange de produits légers distillés en tête de colonne C'1.

### (b) Partie réaction :

- un réacteur de craquage R'1 d'une capacité de 0,5 litre, équipé d'un agitateur mécanique, d'une double enveloppe dans laquelle circule de l'huile à une température régulée, d'une sortie calorifugée en fond de réacteur pour éliminer le résidu de la réaction de craquage, d'une sonde de température. Ce réacteur est surmonté d'une colonne calorifugée de faible hauteur pourvue de pointes Vigreux pour éviter les entraînements de phase liquide (dévésiculeur), et d'un condenseur, après dévésiculeur, alimenté par de l'eau à 20°C. Il est alimenté en continu par le produit obtenu en pied de colonne C'1 ;
- une recette calorifugée, qui reçoit le résidu obtenu en pied du réacteur de craquage R'1 ;
- une recette qui reçoit le mélange de légers condensé en tête du réacteur R'1.

Les conditions opératoires et résultats obtenus lors de la simulation expérimentale de l'étape de distillation sont résumés dans le Tableau 8. Ceux de l'étape de réaction de craquage sont rassemblés dans le Tableau 9.

**Tableau 8**

| Conditions opératoires et résultats de l'étape de distillation | | |
|---|---|---|
| Conditions opératoires | | |
| Débit d'alimentation en g/h | 200 | 250 |
| Débit de distillation en g/h | 122 | 175 |
| Pression en Pa (mm Hg) | 3,7x10³ (50) | 1,9x10⁴ (140) |
| Température tête C'1 en °C | 59 | 70 |

| Compositions C'1 : | | |
|---|---|---|
| - LAA (%) | 41,6 | 26 |
| - LAE (%) | 41,7 | 62 |
| - Légers R'1 (%) | 16,7 | 12 |

| Analyses des flux | | |
|---|---|---|
| Analyses de l'alimentation C'1 : | | |
| - AA (%) | 47,2 | 47,3 |
| - Dimère de l'AA (%) | 13,2 | 5,1 |
| - Trimère de l'AA (%) | 1,2 | |
| - AE (%) | 3,8 | 10,8 |
| - EPE (%) | 4,6 | 6,3 |
| - H₂O (%) | 2,2 | 2,6 |
| - Hydroquinone (%) | 2,3 | |

| Analyses de la tête de C'1 : | | |
|---|---|---|
| - AA (%) | 87,1 | 74,6 |
| - AE (%) | 3,8 | 15 |
| - Ethanol (%) | 1,3 | 6,3 |
| - EPE (%) | 2 | <0,1 |
| - H₂O (%) | 2,8 | 4 |

| Analyses du pied de C'1 : | | |
|---|---|---|
| - AA (%) | 4 | 5,6 |
| - Dimère de l'AA (%) | 25,7 | 18,6 |
| - Trimère de l'AA (%) | 2,9 | 2,4 |
| - AE (%) | <0,2 | <0,2 |
| - Hydroquinone (%) | 4,8 | 3,4 |

**Tableau 9**

| Conditions opératoires et résultats de l'étape de craquage Conditions opératoires | | |
|---|---|---|
| Température R'1 (°C) | 218 | 218 |
| Temps séjour R'1 (min.) | 105 | 97 |
| Taux de craquage (%) | 81 | 79 |

| Analyses des flux (%) | | |
|---|---|---|
| Analyses de l'alimentation de R'1: | | |
| - AA | 4 | 5,6 |
| - Dimère de l'AA | 25,7 | 18,6 |
| - Trimère de l'AA | 2,9 | 2,4 |
| - AE | <0,2 | <0,2 |
| - Hydroquinone | 4,8 | 3,4 |

| Analyses du distillat de R'1 : | | |
|---|---|---|
| - AA | 54,7 | 44,6 |
| - AE | 9,7 | 10,6 |
| - Ethanol | 1,4 | 1,2 |
| - EPE | 12,7 | 21,7 |
| - H₂O | 3,1 | 2,6 |

## Revendications

1. Procédé de récupération des produits nobles légers contenus dans les résidus de distillation des procédés de fabrication de l'acide acrylique et des esters dudit acide et d'alcools en C₁-C₈, par craquage thermique desdits résidus, lesdits produits nobles étant constitués par l'acide acrylique monomère, lesdits esters acryliques monomères et lesdits alcools, caractérisé par le fait qu'on opère ce craquage, en l'absence de catalyseur, sur un mélange de résidus lourds de distillation issus, d'une part, de la fabrication de l'acide acrylique, et d'autre part, de la fabrication des esters précités, qu'on vaporise continuellement, pendant l'opération de craquage, la fraction légère issue des réactions de dissociation, et qu'on distille cette fraction légère afin de récupérer, après condensation, les produits nobles légers recherchés.

2. Procédé selon la revendication 1, caractérisé par le fait que le rapport massique des résidus lourds issus du procédé de fabrication de l'acide acrylique aux résidus lourds issus du procédé de fabrication d'un ester de l'acide acrylique est compris entre 9/1 et 1/9.

3. Procédé selon l'une des revendications 1 et 2, caractérisé par le fait que l'ester est l'acrylate de méthyle ou d'éthyle.

4. Procédé selon l'une des revendications 1 à 3, caractérisé par le fait que l'on conduit le craquage à une température comprise entre 140 et 260°C, avec un temps de séjour de 0,5 à 3 heures.

5. Procédé selon l'une des revendications 1 à 4, caractérisé par le fait que l'on conduit sous pression réduite la distillation de la fraction légère vaporisée.

6. Procédé selon l'une des revendications 1 à 5, caractérisé par le fait que l'on conduit la distillation en présence d'au moins un inhibiteur de polymérisation, le ou les inhibiteurs de polymérisation pouvant être introduits via l'introduction d'une partie du flux alimentant la colonne de distillation.

7. Procédé selon l'une des revendications 1 à 6, caractérisé par le fait que l'on recycle les produits nobles légers obtenus à l'unité de fabrication d'ester de laquelle sont issus les résidus lourds utilisés conjointement avec les résidus lourds issus de la fabrication de l'acide acrylique.

8. Procédé selon l'une des revendications 1 à 7, caractérisé par le fait qu'il est conduit en continu et comprend les opérations consistant à :
- conduire le craquage du mélange de résidus lourds à craquer, éventuellement préchauffé, dans un réacteur (R1) ;
- alimenter une colonne de distillation (C1) par les produits légers du craquage ;
- récupérer, en tête de colonne (C1), le mélange de produits nobles légers recherché ;
- recycler dans le réacteur (R1) le flux récupéré en pied de la colonne (C1) ;
- récupérer le résidu lourd du réacteur (R1) et l'adresser à un traitement d'élimination, le cas échéant après l'avoir dilué avec une partie du flux récupéré en pied de la colonne (C1), le reste dudit flux étant recyclé dans le réacteur (R1).

9. Procédé selon l'une des revendications 1 à 7, caractérisé par le fait qu'il est conduit en continu et comprend les opérations consistant à :
- adresser à une colonne de distillation (C'1) le mélange des résidus lourds à craquer ;
- récupérer, en tête de colonne (C'1), un mélange de produits nobles légers ;
- adresser à un réacteur de craquage (R'1) le flux obtenu en pied de la colonne (C'1) ;
- recycler à la colonne de distillation (C'1) le mélange des produits légers du craquage ;
- récupérer le résidu lourd du réacteur (R'1) et l'adresser à un traitement d'élimination ultérieur, le cas échéant après l'avoir dilué avec une partie du flux récupéré en pied de la colonne (C'1), le reste dudit flux étant adressé au réacteur de craquage (R'1).

## Claims

1. Process for recovery of the light noble products present in the distillation residues from processes for the manufacture of acrylic acid and of eaters of the said acid and of C₁-C₈ alcohols, by thermal cracking of the said residues, the said noble products consisting of acrylic acid monomer, the said acrylic ester monomers and the said alcohols, characterized in that this cracking is performed, in the absence of catalyst, on a mixture of heavy distillation residues originating, on the one hand, from the manufacture of acrylic acid and, on the other hand, from the manufacture of the abovementioned esters, in that the light fraction originating from the dissociation reactions is continuously vaporized during the cracking operation and in that this light fraction is distilled in order to recover, after condensation, the required light noble products.

2. Process according to Claim 1, characterized in that the mass ratio of the heavy residues originating from the process for the manufacture of acrylic acid to the heavy residues originating from the process for the manufacture of an ester of acrylic acid is between 9/1 and 1/9.

3. Process according to either of Claims 1 and 2, characterized in that the ester is methyl or ethyl acrylate.

4. Process according to one of Claims 1 to 3, characterized in that the cracking is conducted at a temperature of between 140 and 260°C with a residence time of 0.5 to 3 hours.

5. Process according to one of Claims 1 to 4, characterized in that the distillation of the vaporized light fraction is conducted at reduced pressure.

6. Process according to one of Claims 1 to 5, characterized in that the distillation is conducted in the presence of at least one polymerization inhibitor, it being possible for the polymerization inhibitor(s) to be introduced via the introduction of a part of the flow feeding the distillation column.

7. Process according to one of Claims 1 to 6, characterized in that the light noble products obtained are recycled to the ester manufacture unit from which originates the heavy residues employed jointly with the heavy residues originating from the manufacture of acrylic acid.

8. Process according to one of Claims 1 to 7, characterized in that it is conducted continuously and includes the operations consisting in:
- conducting the cracking of the mixture of the heavy residues to be cracked, optionally preheated, in a reactor (R1);
- feeding a distillation column (C1) with the light products of the cracking;
- recovering, at the head of column (C1), the required mixture of light noble products;
- recycling into the reactor (R1) the flow recovered at the foot of the column (C1);
- recovering the heavy residue from the reactor (R1) and sending it to a removal treatment, if appropriate after having diluted it with a part of the flow recovered at the foot of the column (C1), the remainder of the said flow being recycled into the reactor (R1).

9. Process according to one of Claims 1 to 7, characterized in that it is conducted continuously and includes the operations consisting in:
- sending the mixture of the heavy residues to be cracked to a distillation column (C'1);
- recovering, at the head of column (C'1), a mixture of light noble products;
- sending the flow obtained at the foot of column (C'1) to a cracking reactor (R'1);
- recycling the mixture of the light products of cracking to the distillation column (C'1);
- recovering the heavy residue from the reactor (R'1) and sending it to a subsequent removal treatment, if appropriate after having diluted it with a part of the flow recovered at the foot of the column (C'1), the remainder of the said flow being sent to the cracking reactor (R'1).

## Patentansprüche

1. Verfahren zur Gewinnung von wertvollen niedrigsiedenden Produkten, die in den bei dem Verfahren zur Herstellung von Acrylsäure und Estern von Acrylsäure und C₁₋₈-Alkoholen entstehenden Destillationsrückständen enthalten sind, durch thermisches Cracken der Rückstände, wobei die wertvollen Produkte aus monomerer Acrylsäure, den oben genannten monomeren Acrylsäureestern und den oben genannten Alkoholen bestehen, dadurch gekennzeichnet, daß das Cracken in Abwesenheit eines Katalysators an einem Gemisch von höhersiedenden Destillationsrückständen durchgeführt wird, die einerseits aus der Herstellung von Acrylsäure und andererseits aus der Herstellung der vorgenannten Ester stammen, während des Arbeitsgangs des Crackens die niedrigsiedende Fraktion, die durch Dissoziation gebildet wird, kontinuierlich verdampft wird und die niedrigsiedende Fraktion destilliert wird, um nach Kondensation die gewünschten wertvollen niedrigsiedenden Produkte zu gewinnen.

2. Verfahren nach Anspruch 1, dadurch gekennzeichnet, daß das Masseverhältnis der hochsiedenden Rückstände aus dem Verfahren zur Herstellung von Acrylsäure zu den hochsiedenden Rückständen aus dem Verfahren zur Herstellung eines Acrylsäureesters im Bereich von 9/1 bis 1/9 liegt.

3. Verfahren nach einem der Ansprüche 1 und 2, dadurch gekennzeichnet, daß der Ester das Methylacrylat oder Ethylacrylat ist.

4. Verfahren nach einem der Ansprüche 1 bis 3, dadurch gekennzeichnet, daß das Cracken bei einer Temperatur im Bereich von 140 bis 260 °C mit einer Verweilzeit von 0,5 bis 3 Stunden durchgeführt wird.

5. Verfahren nach einem der Ansprüche 1 bis 4, dadurch gekennzeichnet, daß die Destillation der verdampften niedrigsiedenden Fraktion unter vermindertem Druck durchgeführt wird.

6. Verfahren nach einem der Ansprüche 1 bis 5, dadurch gekennzeichnet, daß die Destillation in Gegenwart mindestens eines Polymerisationsinhibitors durchgeführt wird, wobei der oder die Polymerisationsinhibitoren über die Zufuhr eines Teils des Stroms, welcher die Destillationskolonne speist, zugeführt werden können.

7. Verfahren nach einem der Ansprüche 1 bis 6, dadurch gekennzeichnet, daß die erhaltenen wertvollen niedrigsiedenden Produkte zur Anlage zur Herstellung der Ester zurückgeführt werden, von welcher die höhersiedenden Rückstände stammen, die gemeinsam mit den höhersiedenden Rückständen aus der Herstellung der Acrylsäure verwendet werden.

8. Verfahren nach einem der Ansprüche 1 bis 7, dadurch gekennzeichnet, daß es kontinuierlich durchgeführt wird und Arbeitsgänge umfaßt, die darin bestehen:
- das Cracken des gegebenenfalls vorgewärmten Gemisches der zu crackenden höhersiedenden Rückstände in einem Reaktionsgefäß (R1) durchzuführen;
- die niedrigsiedenden Crackprodukte in eine Destillationskolonne (C1) einzuführen;
- am Kopf der Kolonne (C1) das gewünschte Gemisch der wertvollen niedrigsiedenden Produkte abzunehmen;
- den am Fuß der Kolonne (C1) abgenommenen Strom in das Reaktionsgefäß (R1) zurückzuführen;
- den höhersiedenden Rückstand des Reaktors (R1) abzunehmen und einer Beseitigungsbehandlung zu unterziehen, wobei er gegebenenfalls zuvor mit einem Teil des Stroms verdünnt wird, der am Fuß der Kolonne (C1) abgenommen wird, wobei der übrige Strom in das Reakti
- onsgefäß (R1) zurückgeführt wird.

9. Verfahren nach einem der Ansprüche 1 bis 7, dadurch gekennzeichnet, daß es kontinuierlich durchgeführt wird und Arbeitsgänge umfaßt, die darin bestehen:
- das Gemisch der zu crackenden höhersiedenden Rückstände einer Destillationskolonne (C'1) zuzuführen;
- am Kopf der Kolonne (C'1) ein Gemisch der wertvollen niedrigsiedenden Produkte abzunehmen;
- den am Fuß der Kolonne (C'1) erhaltenen Strom einem Reaktionsgefäß (R'1), in dem das Cracken durchgeführt wird, zuzuführen;
- das Gemisch der niedrigsiedenden Crackprodukte zur Destillationskolonne (C'1) zurückzuführen;
- den höhersiedenden Rückstand des Reaktors (R'1) abzunehmen und in der Folge einer Beseitigungsbehandlung zu unterziehen, wobei er gegebenenfalls zuvor mit einem Teil des Stromes, der am Fuß der Kolonne (C'1) abgenommen wird, verdünnt wird, und wobei der restliche Strom dem Reaktor zum Cracken (R'1) zugeführt wird.
